# EUROPEAN PATENT SPECIFICATION

(11) **EP 0 391 606 B1**
(45) Date of publication and mention of the grant of the patent: **05.07.1995**
(21) Application number: 90303373.6
(22) Date of filing: 29.03.1990
(51) Int. Cl.: C07C 241/02, C07C 243/22

(54) **Process for the production of an aromatic hydrazo compound**
Verfahren zur Herstellung von aromatischen Hydrazo-Verbindungen
Procédé de préparation de composés hydrazo aromatiques

(30) Priority: 05.04.1989 JP 86362/89
(43) Date of publication of application: 10.10.1990
(73) Proprietor: TOYO INK MANUFACTURING CO., LTD., Tokyo (JP)
(72) Inventor: Maki, Hitoshi, c/o Toyo Ink Manufacturing Co. Ltd., Chuo-ku, Tokyo (JP)
(74) Representative: Woods, Geoffrey Corlett

(56) References cited:
- EP-A- 0 000 519
- EP-A- 0 007 972
- US-A- 3 156 724

## Description

This invention relates to a process for the production of a hydrazo compound by catalytic reduction of an aromatic nitro compound with hydrogen.

An aromatic hydrazo compound is generally produced by a zinc dust process, and it is also produced by a glucose, alcoholate, formalin, alkali hydroxide or iron silicate process. Most of these processes comprise two reaction steps: the first step comprises converting an aromatic nitro compound to an azoxy or azo compound and then separating this product by a physical procedure, and the second step comprises reducing the separated product into a hydrazo compound. The production process is therefore complicated. Further, these conventional processes mostly include complicated steps for separation and recovery of decomposition products of the reducing agent itself and often produce waste liquid containing BOD (COD) source substances. Thus, these processes involve difficulties which greatly lower the economy in hydrazo compound production.

U.S. 3,156,724 discloses a one step process for the production of an aromatic hydrazo compound from an aromtic nitro compound. This process uses a reducing catalyst comprising palladium or platinum. When an aqueous solution containing 2 to 20 % by weight of sodium or potassium hydroxide, particularly, an aqueous solution containing 13 to 14 % by weight of sodium hydroxide, is used as a reaction medium, the reducing catalyst is used while an organic solvent, particularly a water-immiscible aromatic hydrocarbon, such as benzene, toluene or xylene, is added dropwise. The temperature is 40 to 100°C, particularly 60 to 70°C, and the hydrogen pressure is about 0.4 to 7.8 bar, particularly 0.75 to 1.8 bar. When 2,2'-dichlorohydrazobenzene is produced from o-chloronitrobenzene, a naphthalene derivative, e.g. naphthoquinone-(1,4) or 2,3-dichloro-naphthoquinone-(1,4) is added to a reaction mixture. It is described that the yield of 2,2'-dichlorohydrazobenzene obtained in this procedure varies between 80 and 85 % and that chlorine elimination scarcely occurs.

However, the above process not only exhibits a decrease in the yield when a noble metal catalyst is re-used from batch to batch, but also requires a longer period of time for the reaction with decreasing activity of the noble metal catalyst. JP-A-24838/1979 describes that the catalyst used in one batch shows a decrease in activity when a naphthoquinone compound is used as a cocatalyst. These two results are undesirable to carry out the reduction economically. The chlorine elimination amount of 8 % mentioned therein is also undesirably high for a first time use.

Further, JP-A-24838/1979 describes the following. The catalyst can be used for at least ten batches without a decrease in activity and the yield is 83 to 84 % at a lower temperature when o-chloronitrobenzene is catalytically reduced into 2,2'-dichlorohydrazobenzene with hydrogen in a sodium hydroxide aqueous solution or a potassium hydroxide aqueous solution, particularly 10 to 25 wt.% sodium hydroxide aqueous solution in the presence of a water-immiscible aromatic solvent, particularly a hydrocarbon such as benzene, toluene or xylene, by using a noble metal catalyst, particularly, palladium, platinum, or modified or sulfidation one (according to DE 1,959,578), particularly a sulfidation platinum carbon support catalyst (according to DE 2,105,780) under a hydrogen pressure of 1 to about 10 bar, particularly 6 bar and at a reduction temperature of about 50 to 80°C, particularly at 60°C, and, by using, as a cocatalyst, an anthraquinone derivative, particularly a hydroxyanthraquinone such as β-hydroxyanthraquinone or 2,6-dihydroxyanthraquinone.

The process of JP-A-24838/1979 contributes not to the yield but to a decrease in the chlorine elimination ratio. That is, it is reported that the chlorine elimiation ratio is not more than 4 % with palladium, not more than 2 % with a nonmodified platinum and not more than 1 % with sulfidation platinum. However, this process does not satisfactorily decrease the chlorine elimination ratio, nor does it give a satisfactory yield.

It is an object of this invention to provide a process for the production of an aromatic hydrazo compound, which is economical as compared with any conventional process for the production of an aromatic hydrazo compound.

It is another object of this invention to provide a process for the production of an aromatic hydrazo compound, which can achieve high purity and high yield of the aromatic hydrazo compound as compared with any conventional process.

It is further another object of this invention provide a process for the production of an aromatic hydrazo compound, which can reduce the amount of an aniline derivative as a by-product.

It is yet another object of this invention to provide a process for the production of an aromatic hydrazo compound, in which the decrease in the activity of a noble metal catalyst is low even if it is repeatedly used.

It is still another object of this invention to provide a process for the production of an aromatic hydrazo compound, in which the chlorine elimination amount is small even when a chlorine-substituted aromatic nitro compound is used as a raw material.

It is further another object of this invention to provide a process for the production of an aromatic hydrazo compound, in which the amount of hydrogen required for catalytic reduction is reduced.

According to this invention, there is provided a process for the production of an aromatic hydrazo compound, which comprises carrying out catalytic reduction of an aromatic nitro compound with hydrogen in an alkali metal hydroxide aqueous solution in the presence of a noble metal catalyst at a high temperature under a high pressure, wherein the catalytic reduction is carried out by using, as a hydrogen-donating solvent, a cyclic hydrocarbon having 2 to 4 rings and at least two fully saturated carbon atoms within a ring.

The aromatic nitro compound of this invention means compounds having 1 to about 3 aromatic rings, such as nitrobenzene, nitronaphthalene, nitroanthracene, etc. And, 1 to 3 halogen groups, carbonyl groups, nitro groups, amino groups, hydroxyl groups, alkyl groups having 1 to 10 carbon atoms or alkenyl groups having 1 to 10 carbon atoms may be optionally substituted in substitutable positions of the rings.

The alkali metal aqueous solution used in this invention means an aqueous solution of sodium hydroxide or potassium hydroxide, and it particularly means an aqueous solution containing 10 to 25 % by weight of sodium hydroxide.

The noble metal catalyst means a catalyst prepared by allowing an activated carbon to support palladium or platinum in the presence of hydrogen, and the activated carbon/noble metal weight ratio is 1/0.05:

The hydrogen-donating solvent in this invention means a cyclic hydrocarbon formed of 1 to 4 aromatic rings as a basic skeleton and having hydrogen atoms attached to at least two places of the aromatic ring(s). Specifically, it means a cyclic hydrocarbon having a basic skeleton of naphthalene, anthracene, triphenylene, pyrene, chrysene or naphthacene and having 2 to 18 hydrogen atoms attached. More specifically, its examples are 1,2-dihydronaphthalene, 1,4-dihydronaphthalene, tetralin (1,2,3,4-tetrahydronaphthalene), 1,2,3,4,5,6-hexahydronaphthalene, 1,2,3,4,5,8-hexahydronaphthalene, decalin (decahydronaphthalene), 1,2-dihydroanthracene, 1,4-dihydroanthracene, 2,3-dihydroanthracene, 9,10-dihydroanthracene, 1,2,3,4-tetrahydroanthracene, 1,2,7,8-tetrahydroanthracene, 1,2,9,10-tetrahydroanthracene, 2,3,9,10-tetrahydroanthracene, 2,3,8,9-tetrahydroanthracene, 1,2,3,4,5,6-hexahydroanthracene, 1,2,3,4,5,8-hexahydroanthracene, 1,2,3,4,9,10-hexahydroanthracene, 1,4,5,8,9,10-hexahydroanthracene, 1,2,7,8,9,10-hexahydroanthracene, 2,3,6,7,9,10-hexahydroanthracene, 1,2,3,4,5,6,7,8-octahydroanthracene, tetradecahydroanthracene, 1,2-dihydrophenanthrene, 2,3-dihydrophenanthrene, 1,4-dihydrophenanthrene, 9,10-dihydrophenanthrene, 1,2,3,4-tetrahydrophenanthrene, 1,2,5,6-tetrahydrophenanthrene, 1,2,7,8-tetrahydrophenanthrene, 1,2,9,10-tetrahydrophenanthrene, 2,3,6,7-tetrahydrophenanthrene, 3,4,9,10-tetrahydrophenanthrene, 3,4,5,6-tetrahydrophenanthrene, 1,2,3,4,5,6-hexahydrophenanthrene, 1,2,3,4,9,10-hexahydrophenanthrene, 1,4,5,8,9,10-hexahydrophenanthrene, 1,2,7,8,9,10-hexahydrophenanthrene, 2,3,6,7,9,10-hexahydrophenanthrene, 1,2,3,4,5,6,7,8-octahydrophenanthrene, tetradecahydrophenanthrene, 1,2-dihydrotriphenylene, 2,3-dihydrotriphenylene, 1,4-dihydrotriphenylene, 1,2,3,4-tetrahydrotriphenylene, 1,2,5,6-tetrahydrotriphenylene, 1,2,9,10-tetrahydrotriphenylene, 1,2,3,4,5,6-hexahydrotriphenylene, 1,4,5,8,9,12-hexahydrotriphenylene, 2,3,6,7,10,11-hexahydrotriphenylene, 1,2,3,4,5,6,7,8-octahydrotriphenylene, octadecahydrotriphenylene, 1,2-dihydropyrene, 1,2,4,5-tetrahydropyrene, 1,2,6,7-tetrahydropyrene, 1,2,7,8-tetrahydropyrene, 1,2,9,10-tetrahydropyrene, 2,3,4,5-tetrahydropyrene, hexadecahydropyrene, 1,2-dihydrochrysene, 1,2,3,4-tetrahydrochrysene, 1,2,5,6-tetrahydrochrysene, 1,2,8,9-tetrahydrochrysene, 1,2,11,12-tetrahydrochrysene, 1,2,3,4,7,8,9,10-decahydrochrysene, octadecahydrochrysene, 1,2-dihydronaphthacene, 5,12-dihydronaphthacene, 1,2,3,4-tetrahydronaphthacene, 1,2,7,8-tetrahydronaphthacene, 2,3,8,9-tetrahydronaphthacene, 1,4,7,10-tetrahydronaphthacene, 5,6,11,12-tetrahydronaphthacene, 1,2,3,4,5,6,11,12-octahydronaphthacene, octadecahydronaphthacene, etc.

A quinoid compound, which is used as a cocatalyst in this invention, means a compound formed of 1 to 3 aromatic rings as a basic skeleton and having no substituent or having one chlorine atom or hydroxyl group substituted in its substituable position or having two chlorine atoms or two hydroxyl groups substituted in two α-positions, two β-positions or two γ-positions. Its specific examples are o-benzoquinone, 3-chloro-1,2-benzoquinone, 3,-hydroxy-1,2-benzoquinone, 4-chloro-1,2-benzoquinone, 4-hydroxy-1,2-benzoquinone, 3,6-dichloro-1,2-benzoquinone, 3,6-dihydroxy-1,2-benzoquinone, 2,4-dichloro-1,2-benzoquinone, 2,4-dihydroxy-1,2-benzoquinone, p-benzoquinone, 2,5-dichloro-1,4-benzoquinone, 2,5-dihydroxy-1,4-benzoquinone, 2,6-dichloro-1,4-benzoauinone, 2,6-dihydroxy-1,4-benzoquinone, 1,2-napthoquinone, α-chloro-1,2-naphthoquinone, α-hydroxy-1,2-naphthoquinone, β-chloro-1,2-naphthoquinone, β-hydroxy-1,2-naphthoquinone, 4,5-dichloro-1,2-naphthoquinone, 4,5-dihydroxy-1,2-naphthoquinone, 6,7-dichloro-1,2-naphthoquinone, 6,7-dihydroxy-1,2-naphthoquinone, 1,4-naphthoquinone, α-chloro-1,4-naphthoquinone, α-hydroxy-1,4-naphthoquinone, β-chloro-1,4-naphthoquinone, β-hydroxy-1,4-naphthoquinone, 2,3-dichloro-1,4-naphthoquinone, 2,3-dihydroxy-1,4-naphthoquinone, 5,8-dichloro-1,4-naphthoquinone, 5,8-dihydroxy-1,4-naphthoquinone, 2,6-naphthoquinone, α-chloro-2,6-naphtoquinone, α-hydroxy-2,6-naphthoquinone, β-chloro-2,6-naphthoquinone, β-hydroxy-2,6-naphthoquinone, 1,4-dichloro-2,6-naphthoquinone, 1,4-dihydroxy-2,6-naphthoquinone, 3,7-dichloro-2,6-naphthoquinone, 3,7-dihydroxy-2,6-naphthoquinone, 9,10-anthraquinone, α-chloro-9,10-anthraquinone, α-hydroxy-9,10-anthraquinone, β-chloro-9,10-anthraquinone, β-hydroxy-9,10-anthraquinone, 2,3-dichloro-9,10-anthraquinone, 2,3-dihyddroxy-9,10-anthraquinone, 2,6-dichloro-9,10-anthraquinone, 2,6-dihydroxy-9,10-anthraquinone, 1,4-dichloro-9,10-anthraquinone, 1,4-dihydroxy-9,10-anthraquinone, 1,5-dichloro-9,10-anthraquinone, 1,5-dihydroxy-9,10-anthraquinone, 1,2-anthraquinone, α-chloro-1,2-anthraquinone, α-hydroxy-1,2-anthraquinone, β-chloro-1,2-anthraquinone, β-hydroxy-1,2-anthraquinone, 3,7-dichloro-1,2-anthraquinone, 3,7-dihydroxy-1,2-anthraquinone, 3,6-dichloro-1,2-anthraquinone, 3,6-dihydroxy-1,2-anthraquinone, 4,5-dichloro-1,2-anthraquinone, 4,5-dihydroxy-1,2-anthraquinone, 4,8-dichloro-1,2-anthraquinone, 4,8-dihydroxy-1,2-anthraquinone, 1,4-anthraquinone, α-chloro-1,4-anthraquinone, α-hydroxy-1,4-anthraquinone, β-chloro-1,4-anthraquinone, β-hydroxy-1,4-anthraquinone, γ-chloro-1,4-anthraquinone, γ-hydroxy-1,4-anthraquinone, 5,8-dichloro-1,4-anthraquinone, 5,8-dihydroxy-1,4-anthraquinone, 2,3-dichloro-1,4-anthraquinone, 2,3-dihydroxy-1,4-anthraquinone, 2,6-dichloro-1,4-anthraquinone, 2,6-dihydroxy-1,4-anthraquinone, 6,7-dichloro-1,4-anthraquinone, 6,7-dihydroxy-1,4-anthraquinone, 9,10-dichloro-1,4-anthraquinone, 9,10-dihydroxy-1,4-anthraquinone, etc.

The process for the production of an aromatic hydrazo compound of this invention will be explained hereinbelow.

A hydrogen-donating solvent in an aromatic nitro compound/hydrogen-donating solvent weight ratio of from 0.8/1 to 6.3/1, a noble metal catalyst in an aromatic nitro compound/noble metal catalyst weight ratio of from 42,000/1 to 1,500/1, a cocatalyst in an aromatic nitro compound/cocatalyst ratio of 100/1 to 300/1 and an aromatic nitro compound were charged into an ordinary autoclave. Preferably 1 part by weight emulsifier per 100 to 600 parts by weight aromatic nitro compounds may be added. Nitrogen is substituted in the autoclave for air, and further, hydrogen is substituted in the autoclave until the hydrogen initial pressure becomes 3 to 10 kg/cm². The mixture is heated to 50 to 120°C while it is stirred. During the reaction, hydrogen is always supplied into the autoclave from the outside of the reaction system such that the pressure inside the autoclave is the same as the pressure shown immediately after the reaction initiation. When the reaction finishes, the pressure inside the autoclave and the reaction temperature are increased by 10 to 25 percent of those shown at the reaction initiation time. After the reduction, the catalyst is separated by filtration while taking care not to bring it into contact with air, and the separated catalyst is recycled into a next batch without purifying it. An aqueous phase is separated from the reaction solution containing a formed aromatic hydrazo compound and an aniline derivative, then the aniline derivative is removed by washing with diluted hydrochloric acid, the solvent is distilled off, and the aromatic hydrazo compound is dried to measure its yield. Since the formed product has a sufficient purity, it is used for direct conversion into benzidines with a mineral acid. The alkali metal is used in a form of an aqueous solution containing preferably 10 to 25 % by weight of sodium hydroxide or potassium hydroxide.

The emulsifier used in this invention means an anionic or nonionic surfactant, such as an anionic surfactant containing as a main component a fatty acid salt, alkyl sulfate, alkylbenzen sulfonate, alkylnaphthalene sulfonate, dialkyl succinate, alkyl phosphate, formalin naphthalene sulfonate condensate or polyoxyethylene alkyl sulfate, or a nonionic surfactant containing as a main component polyoxyethylene alkyl ether, polyoxyethylene alkyl phenol ether, polyoxyethelene fatty acid ester, sorbitan fatty acid ester, polyoxyethylene sorbitan fatty acid ester or glycerin fatty acid ester.

This invention uses, as a solvent, a hydrogen-donating compound by which the hydrogen transfer can be expected, whereby there is provided a process for the production of a high-purity aromatic hydrazo compound from an aromatic nitro compound at high yields even if a small amount of a catalyst is used.

When a conventional process uses a naphthoquinone-type compound as a cocatalyst, the noble metal catalyst can be hardly used repeatedly, since its activity is degraded when it is used only once. According to this invention, there is provided a process for the production of an aromatic hydrazo compound, in which the noble metal catalyst does not lose its activity and can therefore be used repeatedly even if the naphthoquinone-type compound is used as a cocatalyst. Further, according to this invention, there is provided a process for the production of an aromatic hydrazo compound at a high yield, in which the noble metal catalyst exhibits high activity and is free from a change in reaction time even if the naphthoquinone-type compound is used in the same amount as that of an anthraquinone compound.

The conventional catalytic reduction of o-chloronitrobenzene into 2,2'-dichlorohydrazobenzene has had a problem that chlorine atoms are eliminated during the reaction. According to this invention, there is provided a process for the production of an aromatic hydrazo compound, in which the amount of eliminated chlorine atoms is not more than 1 % by weight of the theoretical value when a palladium catalyst, nonmodified platinum catalyst or the like is used.

According to this invention, there is provided a process for the production of an aromatic hydrazo compound, which is economical since the amount of hydrogen in use is small. This point will be explained below by refering to the use of tetralin as a hydrogen-donating solvent.

During the catalytic reduction, at first, the main reaction uses hydrogen of the hydrogen-donating solvent, tetralin, as well, since it consumes a large amount of hydrogen. Thus, a large part of the tetralin releases hydrogen and changes into naphthalene. And, as the reaction proceeds, the hydrogen consumption amount decreases, and the amount of hydrogen introduced into the reaction system becomes excessive. Hence, the solvent in the form of naphthalene is reduced with hydrogen and changes back to tetralin. Further, due to the presence of a reducing catalyst in the reaction system, part of the tetralin has been further reduced into decalin. Since, however, the amount of the catalyst in the reaction system is not sufficient to reduce all the amount of the detralin, the reduction comes to an end when part of the tetralin becomes decalin, and the hydrogen consumption also stops. This point of time is an end to the main reaction, and when a product is taken and the solvent is recovered, the solvent is a mixture of tetralin with decalin. When this mixture is used as a solvent for a next catalytic reduction reaction, i.e. a second catalytic reduction reaction or that after the second, the hydrogen consumption amount in this catalytic reduction reaction is only about the theoretical amount in the main reaction. If, however, the hydrogen-donating solvent disclosed in this invention is not used, the hydrogen consumption amount in a second catalytic reduction reaction or that after the second is 1.5 times as large as the theoretical amount.

That is, according to this invention, there is provided a process for the production of an aromatic hydrazo compound, in which the hydrogen consumption ammount is small and the yield is high.

According to this invention, there is further provided a process for the production of an aromatic hydrazo compound, in which the amount of aniline derivatives as a by-product is reduced.

### EXAMPLES

This invention will be explained hereinbelow according to Examples, in which "part" stands for "part by weight" and "%" for "% by weight".

### EXAMPLE 1

A 1-liter autoclave having an electromagnetic stirrer, a heating device and a condenser was charged with the following materials.

| | |
|---|---|
| o-Chloronitrobenzene | 315 g (2 moles) |
| Tetralin | 100 ml |
| 25 % Sodium hydroxide aqueous solution | 90 g |
| 2,3-Dichloro-1,4-naphthoquinone | 2.5 g |
| Emulsifier (a commercially available emulsifier mixture containing sodium dodecylbenzene sulfonate as a main component and small amounts of oleic acid, sodium C₁₃-C₁₆ alkylsulfamidocarboxylate and a slightly chlorinated long-chain hydrocarbon) | 1.7 g |
| 5 % Platinum-carbon support catalyst | 0.6 g |

Air within the autoclave closed was fully removed with nitrogen, and then, hydrogen was charged under pressure into the autoclave, with fully removing the nitroten, until the hydrogen pressure became 3 kg/cm². Hydorgen was always introduced to maintain this pressure. Around the time when the reaction finished, the hydrogen pressure was increased up to 6 kg/cm². The reaction temperature was increased, by heating, up to 60°C immediately after the reaction initiation, and when the hydrogen absorption amount decreased, the temperature was increased up to 80°C. The termination of hydrogen absorption was regarded as an end of the reaction. The reduction time was 5 hours. After the reaction, the platinum-carbon support catalyst was thermally filtered from the reaction mixture at 80°C. The filtrate was cooled, and then separated into an organic phase and an aqueous phase. The organic phase (containing 2,2′-dichlorohydrazobenzene and a by-product o-chloroaniline) was washed twice with 5 % hydrochloric acid according to a customary manner to elute o-chloroaniline, and the solvent and a precipitate were separated by filtration. The precipitate was fully washed with methanol and dried. Then, the yield was measured: The yield of 2,2′-dichlorobenzene having a melting point of 85 to 86°C was 91.5 % of the theoretical value and that of o-chloroaniline was 7.5 % (both on the basis of o-chloronitrobenzene).

When the 5 % platinum-carbon support catalyst was used five times, the yields and the reaction times did not vary. The chlorine elimination amount was 0.8 %, at most, of the theoretical value on the basis of o-chloronitrobenzene.

### COMPARATIVE EXAMPLE 1

Example 1 was repeated except that 100 ml of "solvent naphtha" was used in place of the tetralin. The reduction time was 7 hours. The yield was 84.0 % of the theoretical value on the basis of o-chlronitrobenzene, and that of a by-product o-chloroaniline was 10.8 %. The chlorine elimination amount was 4.0 %, at most, on the basis of o-chloronitrobenzene.

When the platinum-carbon support catalyst was repeatedly used, there was a sharp decrease in yield. For example, when it was used for the second time, the reduction time was 12 hours, and the yield was 47.2 % of the theoretical value. The chlorine elimination amount was 5.0 %.

### EXAMPLE 2

According to Example 1, the following materials were reacted.

| | |
|---|---|
| o-Chloronitrobenzene | 315 g (2 moles) |
| Decalin | 100 ml |
| 25 % Sodium hydroxide aqueous solution | 90 g |
| 2,3-Dichloro-1,4-naphthoquinone | 2.5 g |
| Emulsifier (same as that used in Example 1) | 1.7 g |
| 5 % Platinum-carbon support catalyst | 0.6 g |

The reduction time was always 5 hours.

The yield of 2,2'-dichlorohydrazobenzene having a melting point of 85 to 86°C was 85.5 % of the theoretical value on the basis on o-chloronitrobenzene and that of o-chloroaniline was 13.2 %. When the 5 % platinum-carbon support catalyst was used for the fifth time, there was no change in yield and reaction time. The chlorine elimination amount was 1.0 %, at most, of the theoretical value on the basis of o-chloronitrobenzene.

### EXAMPLE 3

Example 1 was repeated except that 0.6 g of a 5 % palladium-carbon support catalyst was used in place of the 5 % platinum-carbon support catalyst. The reduction time was 5 hours.

The yield of 2,2'-dichlorohydrazobenzene having a melting point of 85 to 86°C was 88.9 % of the theoretical value on the basis on o-chloronitrobenzene, and that of a by-product o-chloroaniline was 9.7 %. The chlorine elimination amount was 0.4 %, at most, of the theoretical value on the basis of o-chloronitrobenzene.

### COMPARATIVE EXAMPLE 2

Example 1 was repeated except that 100 ml of "solvent naphtha" was used in place of the tetralin. The reduction time was 8 hours. The yield was 79.5 % of the theoretical value on the basis on o-chloronitrobenzene, and that of a by-product o-chloroaniline was 13.1 %. The chlorine elimination amount was 6.0 %, at most, of the theoretical value on the basis of o-chloronitrobenzene.

### EXAMPLE 4

Example 1 was repeated except that 2.5 g of 2,6-dihydroxylanthraquinone was used in place of 2,3-dichloro-1,4-naphthoquinone. The reduction time was 7 hours.

The yield of 2,2'-dichlorohydrazobenzene having a melting point of 85 to 86°C was 84.9 % of the theoretical value on the basis on o-chloronitrobenzene, and that of a by-product o-chloroaniline was 12.7 %. The chlorine elimination amount was 0.4 %, at most, of the theoretical value on the basis of o-chloronitrobenzene. When the 5 % platinum-carbon support catalyst was used five times, there was no change in yield and reaction time.

### EXAMPLE 5

Example 1 was repeated except that 2,3-dichloro-1,4-naphthoquinone was not used. The reduction time was 8 hours.

The yield of 2,2'-dichlorohydrazobenzene having a melting point of 85 to 86°C was 83.9 % of the theoretical value on the basis on o-chloronitrobenzene, and that of a by-product o-chloroaniline was 9.3 %. The chlorine elimination amount was 0.4 %, at most, of the theoretical value on the basis of o-chloronitrobenzene. When the 5 % platinum-carbon support catalyst was used five times, there was no change in yield and reaction time.

### EXAMPLE 7

Example 1 was repeated except that 100 ml of tetrahydronaphthacene in a molten state was used in place of the tetralin. The reduction time was 12 hours.

The yield of 2,2'-dichlorohydrazobenzene having a melting point of 85 to 86°C was 84.5 % of the theoretical value on the basis on o-chloronitrobenzene, and that of a by-product o-chloroaniline was 9.4 %. The chlorine elimination amount was 0.2 %, at most, of the theoretical value on the basis of o-chloronitrobenzene.

## Claims

1. A process for the production of an aromatic hydrazo compound by the catalytic reduction of an aromatic nitro compound with hydrogen in an alkali metal hydroxide aqueous solution in the presence of a noble metal catalyst at an elevated temperature under an elevated pressure, wherein the catalytic reduction is carried out by using as a hydrogen-donating solvent, a cyclic hydrocarbon having 2 to 4 rings and at least two fully saturated carbon atoms within a ring.

2. A process according to claim 1, wherein the aromatic nitro compound has 1 to 3 aromatic rings.

3. A process according to claim 1 or 2 wherein the aromatic nitro compound has 1 to 3 substituents selected from a hydroxyl group, halogen group, carbonyl group, nitro group, amino group, alkyl group having 1 to 10 carbon atoms and alkenyl group having 1 to 10 carbon atoms.

4. A process according to claim 1, 2 or 3, wherein the alkali metal hydroxide aqueous solution is a sodium hydroxide or potassium hydroxide aqueous solution.

5. A process according to any one of the preceding claims wherein the noble metal catalyst is a platinum-carbon support catalyst or a palladium-carbon support catalyst.

6. A process according to claim 5, wherein the noble metal catalyst has an activated carbon/noble metal weight ratio of 1:0.05.

7. A process according to claim 5 or 6 wherein the aromatic nitro compound/noble metal catalyst weight ratio is from 42,000/1 to 1,500/1.

8. A process according to any one of the preceding claims wherein the cyclic hydrocarbon has a basic skeleton which is that of at least one of naphthalene, anthracene, phenanthrene, triphenylene, pyrene, chrysene and naphthacene and has 2 to 18 fully saturated carbon atoms .

9. A process according to any one of the preceding claims, wherein the catalytic reduction is carried out in the copresence of a quinoid compound.

10. A process according to claim 9, wherein the quinoid compound has a basic skeleton formed of 1 to 3 aromatic rings.

11. A process according to claim 10, wherein the quinoid compound has one or two substituents of chlorine atoms or hydroxyl groups.

12. A process according to claim 10, 11 or 12 wherein the aromatic nitro compound/quinoid compound weight ratio is from 100/1 to 300/1.

13. A process according to any one of the preceding claims wherein the aromatic nitro compound/hydrogen-donating solvent weight ratio is from 0.8/1 to 6.3/1.

## Patentansprüche

1. Verfahren zur Herstellung einer aromatischen Hydrazoverbindung durch die katalytische Reduktion einer aromatischen Nitroverbindung mit Wasserstoff in einer wässrigen Lösung von Alkalimetallhydroxid in Gegenwart eines Edelmetallkatalysators bei einer erhöhten Temperatur unter einem erhöhten Druck, worin die katalytische Reduktion durchgeführt wird, indem als ein Wasserstoff-abgebendes Lösungsmittel ein cyclischer Kohlenwasserstoff mit 2 bis 4 Ringen und mindestens zwei vollständig gesättigten Kohlenstoffatomen innerhalb eines Rings verwendet wird.

2. Verfahren nach Anspruch 1, worin die aromatische Nitroverbindung 1 bis 3 aromatische Ringe aufweist.

3. Verfahren nach Anspruch 1 oder 2, worin die aromatische Nitroverbindung 1 bis 3 Substituenten aufweist, gewählt aus einer Hydroxylgruppe, Halogengruppe, Carbonylgruppe, Nitrogruppe, Aminogruppe, Alkylgruppe mit 1 bis 10 Kohlenstoffatomen und Alkenylgruppe mit 1 bis 10 Kohlenstoffatomen.

4. Verfahren nach Anspruch 1, 2 oder 3, worin die wässrige Lösung von Alkalimetallhydroxid eine wässrige Lösung von Natriumhydroxid oder Kaliumhydroxid ist.

5. Verfahren nach einem der vorangegangenen Ansprüche, worin der Edelmetallkatalysator ein Platin-Kohlenstoff-Trägerkatalysator oder ein Palladium-Kohlenstoff-Trägerkatalysator ist.

6. Verfahren nach Anspruch 5, worin der Edelmetallkatalysator ein Gewichtsverhältnis von aktiviertem Kohlenstoff/Edelmetall von 1:0,05 aufweist.

7. Verfahren nach Anspruch 5 oder 6, worin das Gewichtsverhältnis von aromatischer Nitroverbindung/Edelmetallkatalysator von 42.000/1 bis 1.500/1 beträgt.

8. Verfahren nach einem der vorangegangenen Ansprüche, worin der cyclische Kohlenwasserstoff ein Grundskelett aufweist, das aus mindestens einem von Naphthalen, Anthrazen, Phenanthren, Triphenylen, Pyren, Chrysen und Naphthazen und 2 bis 18 vollständig gesättigten Kohlenstoffatomen besteht.

9. Verfahren nach einem der vorangegangenen Ansprüche, worin die katalytische Reduktion in gleichzeitiger Gegenwart einer chinoiden Verbindung vollzogen wird.

10. Verfahren nach Anspruch 9, worin die chinoide Verbindung ein Grundskelett aufweist, das aus 1 bis 3 aromatischen Ringen gebildet ist.

11. Verfahren nach Anspruch 10, worin die chinoide Verbindung einen oder zwei Substituenten von Chloratomen oder Hydroxylgruppen aufweist.

12. Verfahren nach Anspruch 10, 11 oder 12, worin das Gewichtsverhältnis der aromatischen Nitroverbindung/chinoider Verbindung von 100/1 bis 300/1 beträgt.

13. Verfahren nach einem der vorangegangenen Ansprüche, worin das Gewichtsverhältnis der aromatischen Nitroverbindung/Wasserstoff-abgebendem Lösungsmittel von 0,8/1 bis 6,3/1 beträgt.

## Revendications

1. Procédé de production d'un composé hydrazo-aromatique par la réduction catalytique d'un composé nitro aromatique par de l'hydrogène, dans une solution aqueuse d'un hydroxyde de métal alcalin, en présence d'un catalyseur à base de métal noble à une température élevée sous une pression élevée, procédé dans lequel on effectue la réduction catalytique en utilisant, comme solvant donneur d'hydrogène, un hydrocarbure cyclique comportant 2 à 4 noyaux et au moins 2 atomes de carbone entièrement saturés faisant partie d'un noyau.

2. Procédé selon la revendication 1, dans lequel le composé nitro aromatique comporte 1 à 3 noyaux aromatiques.

3. Procédé selon la revendication 1 ou 2, dans lequel le composé nitro aromatique comporte 1 à 3 substituants, choisis parmi un groupe hydroxyle, un groupe halogéno, un groupe carbonyle, un groupe nitro, un groupe amino, un groupe alkyle ayant 1 à 10 atomes de carbone et un groupe alcényle ayant 1 à 10 atomes de carbone.

4. Procédé selon la revendication 1, 2 ou 3, dans lequel la solution aqueuse d'hydroxyde de métal alcalin est une solution aqueuse d'hydroxyde de sodium ou d'hydroxyde de potassium.

5. Procédé selon l'une quelconque des revendications précédentes, dans lequel le catalyseur à base de métal noble est un catalyseur au platine sur support de charbon, ou de carbone, ou est un catalyseur au palladium sur support de charbon, ou de carbone.

6. Procédé selon la revendication 5, dans lequel le catalyseur à base de métal noble présente un rapport entre pondéral carbone activé et métal noble valant 1: 0,5.

7. Procédé selon la revendication 5 ou 6, dans lequel le rapport pondéral composé nitro aromatique/catalyseur à base de métal noble se situe entre 42000/1 et 150/1.

8. Procédé selon l'une quelconque des revendications précédentes, dans lequel l'hydrocarbure cyclique possède un squelette de base qui est celui d'au moins l'un des systèmes suivants : naphtalène, anthracène, phénanthrène, triphénylène, pyrène, chrysène et naphtacène, et il comporte 2 à 18 atomes de carbone entièrement saturés.

9. Procédé selon l'une quelconque des revendications précédentes dans lequel la réduction catalytique est effectuée en la co-présence d'un composé quinonique.

10. Procédé selon la revendication 9, dans lequel le composé quinonique a un squelette de base formé de 1 à 3 noyaux aromatiques.

11. Procédé selon la revendication 10, dans lequel le composé quinonique comporte un ou deux substituants formés par des atomes de chlore ou par des groupes hydroxyles.

12. Procédé selon la revendication 10, 11, ou 12, dans lequel le rapport pondéral composé nitro aromatique/composé quinonique vaut de 100/1 à 300/1.

13. Procédé selon l'une quelconque des revendications précédentes, dans lequel le rapport pondéral composé nitro aromatique/solvant donneur d'hydrogène se situe entre 0,8/1 et 6,3/1.
